# EUROPEAN PATENT APPLICATION

(11) **EP 1 674 458 A1**
(43) Date of publication of application: **28.06.2006**
(21) Application number: 04380274.3
(22) Date of filing: 27.12.2004
(51) Int. Cl.: C07D 231/12

(54) **Process for obtaining enantiomers of cizolirtine**

(71) Applicant: LABORATORIOS DEL DR. ESTEVE, S.A., 08041 Barcelona (ES)
(72) Inventor: Torrens Jover, Antoni, 08041 Barcelona (ES); Buschmann, Helmut, 08041 Barcelona (ES); Heller, Detleff, 18055 Rostock (DE); Drexler, Hans Joachim, 18055 Rostock (DE)
(74) Representative: Bernardo Noriega, Francisco

(57) **Abstract**

A process is described for the preparation of a precursor alcohol of Cizolirtine, (±)-2-[phenyl(1-methyl-1*H*-pyrazol-5-yl)methoxy]-*N,N-*dimethylethanamine and its enantiomers, it comprises the asymmetric reduction of a prochiral ketone in the presence of a chiral ruthenium (II) catalyst system comprising at least a bidentate phosphorous-containing ligand and a diamine ligand to render chiral alcohols. The chiral alcohols are further O-alkylated to render the corresponding pharmaceutically active ethanamines.

## Description

### FIELD OF THE INVENTION

The present invention relates to the asymmetric reduction of ketones using chiral catalytic systems to render nonracemic chiral alcohols. More particularly, it relates to a new process for the preparation of the pure enantiomers of an intermediate alcohol which is used in the preparation of Cizolirtine, (±)-2-[phenyl(1-methyl-1*H*-pyrazol-5-yl)methoxy]-*N,N*-dimethylethanamine and its enantiomers, comprising the enantioselective hydrogenation of a prochiral ketone.

### BACKGROUND OF THE INVENTION

The compound (±)-2-[phenyl(1-methyl-1*H*-pyrazol-5-yl)methoxy]-*N,N-*dimethylethanamine, also referred to as (±)-5-[α-(2-dimethylaminoethoxy)benzyl]-1-methyl-1*H*-pyrazole, or Cizolirtine of formula (I) 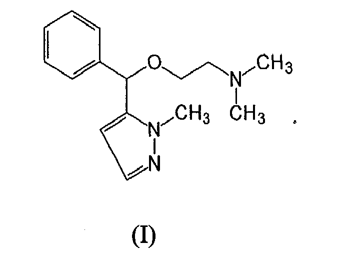 was described in the European patent EP 289 380. This compound is a potent analgesic which is currently in phase II clinical trials.

The two enantiomers of Cizolirtine, hereinafter referred to as (+)-I and (-)-I, have been previously obtained by optical resolution of the Cizolirtine racemate by fractional crystallization with optically active acids (WO 99/02500) such as, for instance, with (-)- and (+)-di-p-toluoyltartaric acid (Torrens, A.; Castrillo, J.A.; Frigola, J.; Salgado, L.; Redondo, *J. Chirality*, 1999, 11, 63). An enantiomerically pure compound synthesis (EPC synthesis) starting from ethyl (R)-mandelate of the intermediate permitted the assignation of the (R) absolute configuration to the (+)-I isomer (Hueso-Rodriguez, J.A.; Berrocal, J.; Gutiérrez, B.; Farré, A..; Frigola, J. *Bioorg. Med. Chem. Lett.* 1993, 3, 269).

The (±)-Cizolirtine has been prepared by O-alkylation of compound (±)-II of formula II:

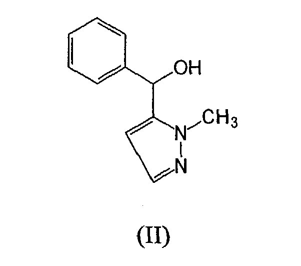

The pure enantiomers of Cizolirtine (+)-I and (-)-I may be prepared by separately O-alkylating the enantiomerically pure intermediates (+)-II and (-)-II.

The enantiomerically pure compounds (+)-II and (-)-II are obtained either by reduction of a compound of formula III, which yields (±)-II as a racemate, followed by procedures of optical resolution of the racemate (±)-II, like fractional recrystallization from solvents or column chromatography [J.A: Hueso, J. Berrocal, B. Gutiérrez, A.J. Farré y J. Frigola, *Bioorg. Med. Chem. Lett.* 1993, 3, 269], or by EPC synthesis starting from the prochiral ketone of formula III:

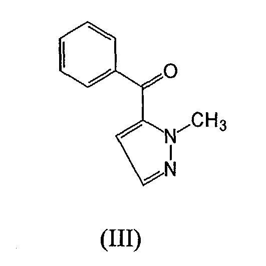

The enantioselective reduction of prochiral ketones in organic synthesis to obtain secondary alcohols with high enantiomeric purity is of high interest since they can be valuable intermediates for the manufacture of active compounds. Accordingly, a number of strategies for the asymmetric reduction of prochiral ketones to single enantiomer alcohols have been developed [R. Noyori, T. Ohkuma, *Angew. Chem. Int. Ed*., 2001, 40, 40-73].

A possibility exists in the use of oxazoborolidines as ligands or catalysts which constitutes a major advance in the asymmetric reduction of prochiral ketones. The use of said chiral ligands or catalysts in combination with achiral reducing agents for the preparation of (+)-I and (-)-I has been described in patent EP 1 029 852 B1. Further examples can be found in the literature [E. J. Corey, Ch. J. Helal, *Tetrahedron Lett.* **1995**, *36*, 9153-9156; E. J. Corey, Ch. J. Helal, *Tetrahedron Lett.* **1996**, *37*, 5675-5678].

However, for diaryl methanols, the reduction of the corresponding ketone precursors is problematic. The chiral catalyst has to differentiate between the two aromatic rings. This can usually only be done with high selectivity if the two rings are different in terms of steric and/or electronic properties which is not obvious in the case of Cizolirtine.

The main strategy for the enantioselective reduction of aromatic and heteroaromatic prochiral ketones with high ee values comprises the use of an optically active diphosphane/Ru/-diamine/inorganic base catalyst system. Moreover, there are also examples with rhodium [K. Kriis, T. Kanger, A.-M. Muurisepp, M. Lopp, *Tetrahedron: Asymmetry* **2003**, 14, 2271-2275.] or cobalt [K. Micskei, C. Hajdu, L. A. Wessjohann, L. Mercs,A. Kiss-Szikszai, T. Patonay, *Tetrahedron: Asymmetry* 2004, 15, 1735-1744] as metall for the asymmetric reduction of prochiral ketones.

Enantioselective hydrogenation of ketonic structures has been achieved with also a wide range of chiral ruthenium catalyst systems which can be prepared by different combinations of Ru (II) chiral phosphanes and diamine ligands. The extent of the enantioselectivity obtained with the different ketones depends largely on the nature of the substituents of the prochiral ketone as shown by the state of the art [see, for instance, Table 2, on p. 53: R. Noyori, T. Ohkuma, Angew. Chem. Int. Ed. 2001, 40, 40-73]. It is also known that heteroaromatic ketones can be enantioselectively hydrogenated to nonracemic secondary alcohols with these chiral ruthenium catalysts systems [C. Chen, R. A. Reamer, J. R. Chilenski, C. J. McWilliams, *Org. Lett*. **2003** 5, 5039;]. One finds further asymmetric hydrogenations with ketones with a phenyl and a heteroaryl in literature [K. Okano, K. Murata, T. Ikariya, *Tetrahedron Lett.* **2000**, *41,* 9277-9280].

However it has been found that one specific catalyst or a class of catalysts cannot be used equally well in all hydrogenations. Thus, to attain satisfactory ee values by the enantioselective hydrogenation of prochiral ketones, each hydrogenation problem has to be investigated separately with regard to the substrate, the catalyst and the reaction conditions for finding the optimal conditions to obtain the best results.

The object of the present invention was to provide a process for the enantioselective hydrogenation of a phenyl pyrazoyl ketone. This process should operate particularly well on industrial scale and be satisfactory as regards yield, conversion and enantiomer excess. In particular the process should be suitable for providing in an advantageous manner specific enantiomer-enriched alcohols as intermediates for the preparation of (+)- and (-)-Cizolirtine.

### SUMMARY OF THE INVENTION

Surprisingly, the inventors have achieved the enantioselective hydrogenation with a chiral ruthenium (II) catalyst system of a prochiral ketone with a phenyl and a methyl-pyrazol substituent comprising two nitrogen atoms, with high ee value and high conversion. Investigations carried out by the inventors have shown in a no way foreseeable manner that the prochiral ketone with a phenyl and a methyl-pyrazol substituent provides the catalytical enantioselective hydrogenation of said ketone with high enantioselectivity and conversion. This could not have been predicted from the nature of the substrate. We have therefore applied this process to the synthesis of the enantiomerically pure intermediates (+)-II and (-)-II and to a process to obtain Cizolirtine and its enantiomers. This process should operate particularly well on an industrial scale and be satisfactory with regard to enantiomer excess, amount and availability of catalyst and in general raw material costs.

Accordingly, the present invention is directed to a process for the preparation of an enantiomerically enriched compound of formula (II): 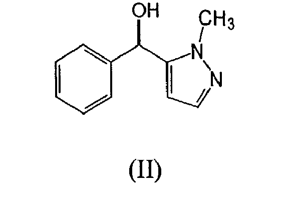 which comprises the asymmetric hydrogenation of a prochiral ketone of formula (III) 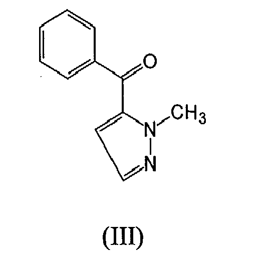 in the presence of a base and a chiral ruthenium (II) catalyst system comprising at least a bidentate phosphorous-containing ligand and a diamine ligand.

Said process allows the preparation of the known intermediates of formula II, which can be optionally transformed in enantiomerically pure pharmaceutically active compounds such as (+)-Cizolirtine and (-)-Cizolirtine.

### DETAILED DESCRIPTION OF THE INVENTION

The process of the invention gives the desired product of formula II with high conversion and enantiomeric excess. This process has the further advantage that the starting materials are not expensive and that it works under low or normal pressures. Similar hydrogenations are known, as mentioned above, but it is the first time they are applied to a pyrazol containing substrate. Although problems due to the coordination of the pyrazol could have been expected, on the contrary we have found that the reaction works remarkably well providing a simple route to the alcohols of formula (II) with high conversion and enantiomeric excess. It allows the compounds of the above formula (II) to be synthesised directly from the compounds of formula (III), without any further intermediate steps or laborious separation of the isomeric forms.

The product of formula II is especially useful in the preparation of Cizolirtine enantiomers. The details of the process are discussed below.

The chiral ruthenium (II) catalyst system used in the process of the present invention is known to the person skilled in the art and is composed of Ruthenium (II) complexes with two different ligands, a bidentate phosphorous-containing ligand and a diamine, in the presence of a base. Said catalyst system components can be provided to the reaction mixture individually to form the reactive catalyst system *in situ* or they can be provided as preformed complexes.

The bidentate phosphorous-containing ligand is in general of the biphosphines or biphosphites types, more preferably it is of the biphosphine type. Illustrative examples of nonracemic chiral diphosphines are 2,2'-bis(diphenyl-phosphino)-1,1'-binaphtyl (BINAP), TolBINAP and XylBINAP [R. Noyori, T. Ohkuma, Angew. Chem. Int. Ed., 2001, 40, 40-73], 2,2'-bis(diphenylphosphino)-1,1'-dicyclopentane (BICP) [P. Cao, X. Zhang, *J. Org. Chem*. **1999** *64*, 2127-2129.], 2,2',6,6'-tetramethoxy-4,4'-bis-3,3'-bipyridine (P-Phos), Tol-P-Phos and Xyl-P-Phos [J. Wu, H. Chen, W. Kwok, R. Guo,Z. Zhou, C. Yeung, A. S. C. Chan, *J*. *Org. Chem*. **2002,** *63,* 7908-7910], 4,12-bis(diphenylphosphino)[2.2]paracyclophane (PhanePhos) and Xyl-PhanePhos [M. J. Burk, W. Hems, D. Herzberg, C. Malan, A. Zanotti-Gerosa, *Org. Lett*. **2000,** *2*, 4173-4176] and equivalents thereto that are recognized by those skilled in the art.

In one preferred embodiment the diphosphine ligand comprises a binaphthyl group. They are more preferably selected from the group consisting of the enantiomers of 2,2'-bis(diphenyl-phosphino)-1,1'-binaphtyl (BINAP), TolBINAP and XylBINAP [see R. Noyori, T. Ohkuma, Angew. Chem. Int. Ed., 2001, 40, 40-73].

Suitable diamines are 1,2-diamine species that exhibit a sufficient activity or selectivity in the catalyst under consideration. They can be chiral or non-chiral. Ilustrative examples are any stereoisomers of 1,1-bis(4-methoxyphenyl)-3-methyl-1,2-butanediamine (DAIPEN), 1,2-diphenylethylendiamine (DPEN), 1,2-diaminocyclohexane (DACH) or achiral diamines such as ethylenediamine. Achiral amines are further discussed in US 6,743,921 which is incorporated herein by reference in its entirety.

The use of enantiomerically enriched diamines such as DAIPEN and DPEN has proved particularly advantageous, preferably is DPEN regards costs and higher activity and selectivity.

The bidentate phosphorous-containing ligand together with the diamine and the ruthenium (II) form a complex referred to hereinafter as the ruthenium (II) component of the catalyst system. Examples of preformed complexes of the ruthenium with the diphosphine ligand and the diamine include complexes represented by the formula RuX₂LA wherein X represents a halogen atom or pseudo-halide group, preferably chloride or bromide, L represents the diphosphine ligand and A is the diamine. Suitable examples are RuCl₂ [(S)-BINAP][(R,R)-DPEN], RuCl₂ [(S)-BINAP][(S,S)-DPEN], RuCl₂ [(R)-BINAP][(R,R)-DPEN], RuCl₂ [(R)-BINAP][(S,S)-DPEN], RuCl₂ [(R)-BINAP][(R)-DAIPEN], RuCl₂ [(S)-BINAP][(S)-DAIPEN].

Said component is present in catalytic amounts, meaning less than stoichiometric relative to the ketone reactants and as low as possible while ensuring the optimum possible conversion rate. The minimum amount of the ruthenium (II) component of the catalyst system may depend on the activity of the specific catalyst system composition, the reaction temperature, the concentration of the reactants and catalyst system components in the solution, the hydrogen pressure and the maximum time allowed for completion of the reaction. In a typical embodiments the molar ratio of the ruthenium (II) component of the catalyst to the ketone reactant (s/c) is in the range from about 50 to 20000, preferably from about 200 to about 20.000, more preferably from about 10.000 to about 20.000.

Suitable bases include organic bases and inorganic bases which should not have a negative influence on, for example, the enantiomer purity of the products that are formed. Preferably, the base is selected from the group consisting of a hydroxide, C₁₋C₅-alkoxide, bicarbonate, carbonate, di- and tribasic phosphate, borate, fluoride, amine optionally substituted with C₁-C₄-alkyl or aryl, silane optionally substituted with C₁-C₃₋alkyl.
In this connection alkali metal alcoholates are advantageous, such as for example *t-*BuOK, as well as inorganic bases such as for example KOH or K₂CO₃. Also used are organic nitrogen bases such as NEt₃ and salts as for example AgCF₃SO₃. In a more preferred embodiment *t*-BuOK is used. When the base used is *t*-BuOK it is preferably added to the reaction vessel in form of a solution of *t*-BuOK in *t*-BuOH.

It has been found that a molar excess of base referred to the ruthenium (II) component of the catalyst system is advantageous. The typical mole ratio base : ruthenium (II) component of the catalyst system is comprised between 10:1 and 1, more preferably between about 4:1 and about 2:1. It has been found that both the activity and the selectivity of the hydrogenation vary with the amount of the base. In this connection the activity of the hydrogenation increases with rising concentration of the base. However, if the concentration of base is too high there is a possibility of racemization of the end product, which is not desirable. A ratio of about 4:1 is particularly preferred.

The hydrogenation reaction is conducted in a solvent system that is capable of dissolving the catalyst system and is reaction-inert. The term solvent system is used to indicate that a single solvent or a mixture of two or more solvents can be used. The term reaction-inert is used to mean that the solvent system does not react unfavourably with the reactants, products, or the catalyst system. The solvent system need not bring about complete solution of the ketone reactant or the chiral alcohol product. The ketone reactant may be incompletely dissolved at the beginning of the reaction or the chiral alcohol product may be incompletely dissolved at the end of the reaction, or both. Representative solvents are alcohol solvents such as methanol, ethanol, n-propanol, 2-propanol, n-butanol, *sec*-butanol or t-butanol and their mixtures, organic solvents containing heteroatoms such as DMF and ethers like THF. Preferably the solvent system comprises an alcohol solvent, more preferably methanol, isopropanol, t-butanol and their mixtures. *Tert*-butanol is particularly preferred.

The hydrogenation takes place in a suitable reactor known to the person skilled in the art, such as an autoclave. It is advisable to carry out the hydrogenation under an inert gas atmosphere. Suitable media are nitrogen gas or a noble gas such as argon.

The temperature during the reaction may in principle be chosen arbitrarily by the person skilled in the art as long as a sufficient quick and selective reaction is guaranteed. However, it has to be taken into account that the temperature depends strongly on solvent and that some catalyst systems are instable above 40 °C. In typical embodiments the reaction is suitably conducted at a temperature comprised between 10 and 45 °C, preferably between 20 and 35 °C, and most preferably about 30 °C.

The hydrogenation refers to reacting the ketone with a source of hydrogen atoms under appropriate conditions so that two hydrogen atoms are added to the carbonyl group of the ketone to produce the hydroxyl group of the chiral alcohol. Preferably the source of hydrogen atoms includes molecular hydrogen (H₂). If the hydrogenation is carried out in the presence of molecular hydrogen, the hydrogen pressure in the reaction is preferably low, typically at least about a 1.3 atm. Normally it is in the range from 0.8 to 100 bar. More typically the hydrogen pressure is in the range from 1.3 to 8 bar.

The ketone of formula (III) is known and can be prepared as described for example in WO99/07684 or any other method readily apparent to the person skilled in the art. Normally the ketone substrate (III), the catalyst system and the base (if it is a solid) are weighted and introduced in the reactor. Then the solvent is added and stirred to complete dissolution of the catalyst. Thereafter the base, if not a solid, is added. The reactor is brought to the adequate temperature and pressure to complete the reaction. Alternatively, the ketone of formula (III) is dissolved in an appropriate solvent, then the constituents of the catalyst system or the catalyst in preformed form are added, and then the hydrogenation is performed at an appropriate temperature and suitable hydrogen pressure.

The ketone concentration ranges from about 0.025 to 0.1 mol/l, preferably from about 0.05 mol/l. In general the reaction is allowed to continue until complete conversion of the ketone. Times comprised between 1 to 110 hours are sufficient, although shorter times are preferred in terms of economy of the process.

The advantages associated with the invention are numerous: The process according to the invention provides a simple means of access to isomers which were previously relatively difficult to obtain, and also allows this to be done on a large industrial scale with excellent productivity. The process according to the invention makes it possible to prepare the desired product not only in high yields but also with very high enantioselectivity. No additional purification steps are needed, the products may be further processed directly just as they occur.

Conversions of 100% of the ketone are achieved by the process of the present invention. The enantiomers proportions achieved by the process of the invention are above 80 ee% and can be as high as 82 ee%. Since the constituents of the catalyst (diamine, ruthenium (II) and bidentate phosphorous containg ligand) may be used in several diasteromeric and enantiomeric forms and the complex in each case may therefore be present in so-called matched or mismatched configurations with regard to the chiral ketone, the person skilled in the art must check which pair works most suitably regards selectivity.

In a preferred embodiment the process is directed to the synthesis of each of the following alcohols of formula II with the highest possible enantiomeric purity:

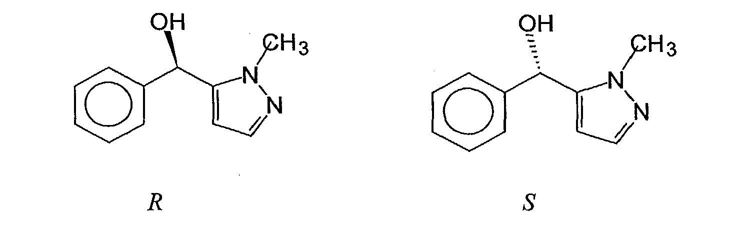

Once the hydrogenation is completed, the obtained hydrogenated product, i.e. the enantiomers of the nonracemic alcohol mixtures [(+)-II and (-)-II], may be subjected to additional purification, for example one or more washing steps and/or recrystallisation from the solvent used, and may be separated off and worked up in the usual way.

Thus, in another aspect, the invention relates to a process as defined above which further comprises the step of O-alkylation of an enantiomerically enriched compound of formula (II) to yield the desired enantiomer of the pharmaceutically active Cizolirtine (I). To this end, the compound of formula (II) is treated with an amine of formula

X-CH₂CH₂N(Me)₂

wherein X is a suitable leaving group such as halogen, more preferably chlorine, bromine or iodine; a reactive esterified hydroxyl, for example arylsulphonyloxy such as phenylsulphonyloxy; tosyloxy; mesyloxy; C₁₋₄ alkyl sulphonyloxy, for example methanesulphonyloxy; arylphosphoryloxy, for example diphenylphosphoryloxy, dibenzylphosphoryloxy or a C₁₋₄ alkyl phosphoryloxy, for example dimethylphosphoryloxy.

An appropriate O-alkylation has been described in EP289 380 or WO 99/07684, the content of these patent applications is incorporated herein in their entirety.

The alkylation is preferably carried out directly in the same reaction medium resulting from the process of the invention, without further purification of the carbinol. Alternatively the solvent can be evaporated and a apolar solvent such as toluene added for the alkylation.

In general, the O-alkylation is carried out in conditions of phase transfer, using for example 2-chloro-*N,N*,-dimethylethylamine (other leaving groups instead of chloro are possible), an alkaline aqueous solution such as NaOH or KOH, in the presence of a catalyst such as a quaternary ammonium salt. Accordingly, the same solvent as the one used in the process of the invention is used, such as toluene. In these conditions we have the further advantage that the impurities like any remaining zinc salts are also eliminated through the aqueous phase.

The resulting product of formula I is enantiomerically enriched, it can be further purified using polar organic solvents. Further, a pharmaceutically acceptable salt of the compound of formula I can be formed. For example, the citrate salt can be prepared by dissolving the amine of formula I in ethanol and treating the solution with citric acid monohydrate. The preparation of other salts will be readily apparent to the person skilled in the art.

The following examples will further illustrate the invention, they should not be interpreted as limiting the scope of the invention.

### EXAMPLES

### General Methods and Materials.

### a) Reactions in autoclave

The substrate, and the components of the chiral ruthenium (II) catalyst system used in the process of the present invention: bidentate phosphorous-containing ligand, amine and base (if the base is a solid) are weighed (not necessarily anaerobic) in a schlenk flask. With larger quantities of substrate (more than 1.5 mmol) the substrate is filled directly into the autoclave. The schlenk flask is securated and the solvent (stock solution) is added under anaerobic conditions. The formed suspension is stirred up to the dissolution of the chiral ruthenium (II) catalyst system (ca. 5 min). Then the base solution is added with a securated Hamilton glass syringe and stirred again 5 min if it was not already added as a solid at the beginning. Afterwards the solution is transferred into the securated autoclave standing under vacuum (via capillary and argon pressure). The reaction solution is then heated up to the desired temperature. The desired hydrogen pressure is adjusted.

### b) Reactions at normal pressure

The substrate, and the components of the chiral ruthenium (II) catalyst system used in the process of the present invention: bidentate phosphorous-containing ligand, amine and base (if the base is a solid) are weighed (not necessarily anaerobic) and given in a temperaturable two neck reaction vessel. This is connected to a dropping funnel containing the solvent (stock solution, anaerobic conditions) and the normal pressure registration equipment. Afterwards this complete system is carefully securated. Furthermore the solution in the dropping funnel is added to the solids in the reaction vessel and the base solution is added to the suspension. Then the argon is replaced with hydrogen (3 x securation with hydrogen). Normal pressure is adjusted by deflating the overpressure over a bubble counter and the measurement is started.

### Example 1. Preparation of the enantiomerically enriched phenyl 1-methylpirazoyl carbinol

**Table 1: Preparation of the enantiomerically enriched phenyl 1-methylpirazoyl carbinol (variation of standard condition*)**

| entry | s/c | Diamine | solvent | pressure temperature | time (conversion) | enantiomeric excess |
|---|---|---|---|---|---|---|
| 1 | 50 | R,R-DPEN | 20 ml MeOH | 100 bar 30 °C | 2 h (100 %) | 42 ee% |
| 2 | 50 | R,R-DPEN | 20 ml MeOH | 100 bar 25 °C | 18 h (100 %) | 53 ee% |
| 3 | 100 | R,R-DPEN | 20 ml isopropanol | 8 bar 35 °C | 3.5 h (100 %) | 73 ee% |
| 4 | 100 | R,R-DPEN | 20 ml isopropanol | 8 bar 30°C | 2 h (100 %) | 79 ee% |
| 5 | 100 | R,R-DPEN | 20 ml isopropanol | 8 bar 25 °C | 5 h (100 %) | 81 ee% |
| 6 | 100 | R,R-DPEN | 20 ml t-butanol | 8 bar 30 °C | 3.5 h (100 %) | 82 ee% |
| 7 | 100 | R,R-DPEN | 19 ml t-butanol 1 ml isopropanol | 8 bar 30 °C | 1 h (100 %) | 80 ee% |
| 8 | 100 | R-DAIPEN | 20 ml isopropanol | 8 bar 25 °C | 3 h (100 %) | 73 ee% |
| 9 | 100 | ethylen- diamine | 20 ml isopropanol | 8 bar 25 °C | 4 h (100 %) | 33 ee% |
| 10 | 100 | R,R-DPEN | 20 ml isopropanol | 1 bar 25 °C | 20 h (98 %) | 75 ee% |
| 11 | 100 | R,R-DPEN | 19 ml t-butanol 1 ml isopropanol | 1 bar 25 °C | 20 h (100 %) | 75 ee% |

| | | | | | | |
|---|---|---|---|---|---|---|
| * Standard conditions: 0.01 mmol *R,R*-Ru(BINAP); 0.01 mol amine; 0.5 or 1 mmol 1-methylpirazoyl carbinol; 0.04 mmol *t*-BuOK; 20 ml solvent; 1 - 100 bar H₂; 10 - 45 °C. | | | | | | |

### Entry 1 and 2

### according to method a)

The compound was prepared from 0.5 mmol phenyl 1-methylpyrazoyl ketone
0.01 mmol *R*-Ru(BINAP); 0.01 mmol *R,R*-DPEN
0.04 mmol *t*-BuOK (40 µl, *t*-BuOK 1.0 M solution in *t*-BuOH);
20 ml methanol,
at 25°C or 30°C and 100 bar H₂.
Conversion: 100 % after 2 h with 42 %ee for 30 °C and
100 % after 18 h with 53 %ee for 25 °C.

### Entry 3, 4 and 5

### according to method a)

The compound was prepared from 1 mmol phenyl 1-methylpyrazoyl ketone
0.01 mmol *R*-Ru(BINAP); 0.01 mmol *R,R*-DPEN
0.04 mmol *t*-BuOK (40 µl, *t*-BuOK 1.0 M solution in *t*-BuOH);
20 ml isopropanol,
at 25°C, 30°C or 35 °C and 8 bar H₂.
Conversion: 100 % after 3.5 h with 73 %ee for 35 °C,
100 % after 2 h with 79 %ee for 30 °C and
100 % after 5 h with 81 %ee for 25 °C.

### Entry 6 and 7

### according to method a)

The compound was prepared from 1 mmol phenyl 1-methylpyrazoyl ketone
0.01 mmol *R*-Ru(BINAP); 0.01 mmol *R,R*-DPEN
0.04 mmol *t*-BuOK (40 µl, *t*-BuOK 1.0 M solution in *t*-BuOH);
20 ml *t*-butanol or 19 ml t-butanol and 1 ml isopropanol;
at 30°C and 8 bar H₂.
Conversion: 100 % after 3.5 h with 82 %ee for 20 ml t-butanol and
100 % after 1 h with 80 %ee for 19 ml t-butanol and 1 ml isopropanol.

### Entry 8 and 9

### according to method a)

The compound was prepared from 1 mmol phenyl 1-methylpyrazoyl ketone
0.01 mmol *R*-Ru(BINAP); 0.01 mmol R-DAIPEN or ethylendiamine;
0.04 mmol *t*-BuOK (40 µl, *t*-BuOK 1.0 M solution in *t*-BuOH);
20 ml isopropanol;
at 25°C and 8 bar H₂.
Conversion: 100 % after 3 h with 73 %ee for DAIPEN and

100 % after 4 h with 33 %ee for ethylendiamin.

### Entry 10 and 11

### according to method b)

The compound was prepared from 1 mmol phenyl 1-methylpyrazoyl ketone
0.01 mmol *R*-Ru(BINAP); 0.01 mmol R,R-DPEN
0.04 mmol *t*-BuOK (40 µl, *t*-BuOK 1.0 M solution in *t*-BuOH);
20 ml isopropanol or 19 ml *t*-butanol and 1 ml isopropanol;
at 25°C and 1 bar H₂.
Conversion: 98 % after 20 h with 75 %ee for 20 ml isopropanol and
100 % after 20 h with 75 %ee for19 ml t-butanol and 1 ml isopropanol.

The best results where obtained with the 19 to 1 mixture of *t*-butanol and isopropanol at 8 bar and 30 °C, see example 7.

## Claims

1. A process for the preparation of an enantiomerically enriched compound of formula (II): 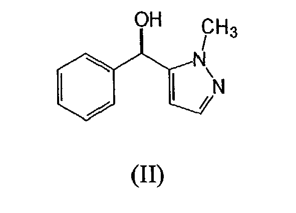 which comprises the asymmetric hydrogenation of a prochiral ketone of formula (III) 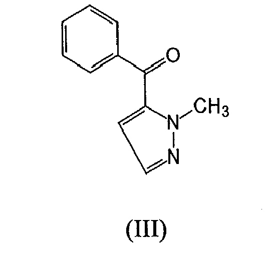 in the presence of a base and a chiral ruthenium (II) catalyst system comprising at least a bidentate phosphorous-containing ligand and a diamine ligand.

2. A process accoding to claim 1, wherein the bidentate phosphorous-containg ligand is a bisphosphine ligand comprising a binaphthyl group, preferably selected from the group formed by the stereoisomers of 2,2'-bis(diphenyl-phosphino)-1,1'-binaphtyl (BINAP), ToIBINAP and XyIBINAP.

3. A process according to claim 1 wherein the diamine is an enantiomerically-enriched 1,2 diamine, preferably selected from the group consisting of 1,1-bis(4-methoxyphenyl)-3-methyl-1,2-butanediamine (DAIPEN), 1,2-diphenylethylendiamine (DPEN) or 1,2-diaminocyclohexane (DACH).

4. A process according to claim 3 wherein the diamine is DPEN.

5. A process according to claim 1 wherein the base is selected from alkali metal alcoholates, preferably *t*-butanolate.

6. A process according to claim 1 wherein the base is selected from *t*-BuOK, KOH, K₂CO₃, NEt₃ and AgCF₃SO₃, preferably *t*-BuOK.

7. A process according to claim 1 wherein the mole ratio base : ruthenium (II) component of the catalyst system is comprised between 10:1 and 1:1, preferably between 4:1 and 1:1, more preferably between about 4:1 and 2:1.

8. A process according to claim 1 wherein the solvent is an alcohol, preferably selected from methanol, isopropanol, t-butanol and their mixtures, more preferably is *t*-butanol.

9. A process according to claim 1 which further comprises an O-alkylation of the enantiomerically enriched compound of formula II to prepare respectively (+)-Cizolirtine and (-)-Cizolirtine.

10. A process according to claim 9 wherein the O-alkylation is carried out on the product of the process as defined in anyone of claims 1-8, without an intermediate separation or purification step.
